# EUROPEAN PATENT APPLICATION

(11) **EP 1 970 047 A1**
(43) Date of publication of application: **17.09.2008**
(21) Application number: 07251080.3
(22) Date of filing: 15.03.2007
(51) Int. Cl.: A61K 9/10, A61K 31/573, A61P 17/00

(54) **Polyaphron topical composition with a corticosteroid**

(71) Applicant: Drug Delivery Solutions Limited, Leatherhead, Surrey KT22 7RY (GB)
(72) Inventor: Wheeler, Derek Alfred, Leatherhead Surrey KT22 7RY (GB); Georgiou, Michelle, Leatherhead Surrey KT22 7RY (GB)
(74) Representative: Hayes, Adrian Chetwynd

(57) **Abstract**

A topical composition comprising a continuous phase and at least one discontinuous phase, said composition comprising at least one polyaphron dispersion and at least one corticosteroid and said continuous phase comprising 0 to 60% by weight of a C₁ - C₄ alcohol, ethylene glycol, a liquid polyethylene glycol, propylene glycol, a liquid polypropylene glycol, diethylene glycol mono ethyl ether or mixtures thereof.

## Description

The present invention relates to a composition for topical application comprising at least one corticosteroid.

It is known for compositions comprising steroids to be used for the treatment of a number of skin conditions, for example radiation dermatitis.

Further, it is known in the prior art to treat a number of skin conditions by applying a combination of two or more pharmacologically active compounds. For example, in the treatment of psoriasis, it is possible to use a combination treatment involving a vitamin D analogue, such as calcipotriol, and a steroid, such as corticosteroid, where each of the active compounds is formulated in a separate preparation (for example in US-B-6,753,013).

US-B-6,753,013 discloses a pharmaceutical composition for dermal use, which contains at least one vitamin D or vitamin D analogue, at least one corticosteroid and a solvent selected to enable the two active components to coexist without significant degradation, despite their different stability profiles. The compositions, however, are wax-based and include wax or similar excipients, such as soft white paraffin and paraffin liquid. A disadvantage of this composition is that, in order to mix the vitamin D or vitamin D analogue and the corticosteroid into the wax, the wax must be heated to a temperature of 70°C. Such elevated temperatures may damage the drugs in the composition. Furthermore, wax based compositions tend to be rather oily, which after application leave a greasy film on the skin. This is undesirable.

There is a need to formulate an improved composition suitable for topical application which addresses at least some of the problems of the prior art.

US-A-2006/0239947 discloses a biliquid foam comprising 10 to 98% by weight of a non-polar liquid and 2 to 88% by weight of a continuous phase polar liquid comprising a C₁-C₄ alcohol, a liquid polyethylene glycol, ethylene glycol or propylene glycol in an amount of at least 65% by weight relative to the weight of the continuous phase.

The present inventors have now developed a new composition comprising at least one corticosteroid. The present inventors have surprisingly found that such compositions have an enhanced dermal diffusion rate and/or improved stability compared to known compositions. Such compositions also have an appropriate viscosity such that they are useful for topical application.

US-A-2006/0228408 discloses an oral drug delivery system comprising a biliquid foam which comprises a poorly water soluble drug such as a corticosteroid. The composition disclosed is suitable for oral delivery for which purpose the level of continuous phase, typically water, must be kept to a minimum.

Accordingly, the present invention provides a topical composition comprising a continuous phase and at least one discontinuous phase, said composition comprising at least one polyaphron dispersion and at least one corticosteroid and said continuous phase comprising from 0 to 60% by weight of a C₁ - C₄ alcohol, ethylene glycol, a liquid polyethylene glycol, propylene glycol, a liquid polypropylene glycol, diethylene glycol mono ethyl ether or mixtures thereof.

According to another aspect of the present invention there is provided a composition as described herein for use in the treatment of radiation dermatitis.

According to another aspect of the present invention there is provided a composition as described herein for use in the treatment of eczema.

According to another aspect of the present invention there is provided a composition as described herein for use in the treatment of rheumatoid arthritis.

According to another aspect of the present invention there is provided a composition as described herein for use in the manufacture of a medicament for the treatment of radiation dermatitis.

According to another aspect of the present invention there is provided a composition as described herein for use in the manufacture of a medicament for the treatment of eczema.

According to another aspect of the present invention there is provided a composition as described herein for use in the manufacture of a medicament for the treatment of rheumatoid arthritis.

According to a further aspect the present invention provides a composition as described herein for use in a method of treatment of the human or animal body by therapy.

According to a further aspect there is provided a method of treatment or prophylaxis of radiation dermatitis in a subject which comprises topically applying to a subject an effective amount of a composition as herein described.

According to a further aspect there is provided a method of treatment or prophylaxis of eczema in a subject which comprises topically applying to a subject an effective amount of a composition as herein described.

According to a further aspect there is provided a method of treatment or prophylaxis of rheumatoid arthritis in a subject which comprises topically applying to a subject an effective amount of a composition as herein described.

In the following description, the meaning of the terms used are as follows: by hydrophilic phase or solvent is meant a liquid phase comprising water, comprising water together with other water-miscible liquids, or comprising a non-aqueous liquid which is miscible with water. By hydrophobic phase or solvent is meant a phase comprising pharmaceutically acceptable liquids such as oils that are immiscible or substantially immiscible with the hydrophilic phase. By immiscible liquids is meant that when mixed together, they separate to form two distinctly separate liquid phases sharing a well-defined interface. By substantially immiscible is meant that two liquids mixed as above having a well-defined interface between two phases where each phase may nevertheless contain small quantities of dissolved molecules of the other phase.

According to another aspect of the present invention there is provided a method of making the composition as described herein comprising the following steps:
(i) providing a hydrophilic solvent, optionally comprising at least one corticosteroid, and/or a surfactant;
(ii) providing a hydrophobic solvent optionally comprising at least one corticosteroid, and/or a surfactant;
(iii)mixing the hydrophilic solvent with the hydrophobic solvent under suitable conditions to form the composition comprising at least one polyaphron dispersion and at least one corticosteroid.

Advantageously, the compositions of the present invention have an enhanced dermal permeation of the active agent compared to commercially available compositions. Thus, lower levels of active agents may be required in the compositions of the present invention in order to achieve beneficial treatment results. As a result of having lower levels of corticosteroid in the compositions of the present invention the likelihood of causing skin irritation is reduced.

Advantageously, in the compositions of the present invention the corticosteroid can exist in a predominantly non-aqueous environment, having the appropriate and controllable viscosity due to the presence of polyaphron dispersions together with any gelling agents included in the composition. A further particular advantage is that the compositions have good long term stability even at elevated temperature (40°C). Preferably the compositions may contain water. This may be useful for dissolving water-soluble additives such as water-soluble preservatives, antioxidants, water-soluble permeation enhancers and the like.

A further advantage is that the compositions of the present invention are typically manufactured at room temperature without the need to apply heat, making it less likely that actives will be damaged in the composition.

A further advantage of the present composition is that it feels less greasy in use compared to, for example, the compositions of US-B-6,753,013, making it more pleasant to apply and thereby increasing the likelihood of proper patient compliance.

A yet further advantage of the composition of the present invention is that it need not comprise a high level of surfactant. In high concentrations surfactants are known to cause skin irritation. It is therefore desirable to keep the surfactant level to a minimum when applied to skin. Preferably the compositions of the present invention comprise less than 4% by weight of surfactant, more preferably less than 3%, more preferably still less than 2% by weight of the total composition.

By polyaphron dispersion as used herein is meant a particular kind of hydrophilic liquid in hydrophobic liquid or hydrophobic liquid in hydrophilic liquid dispersion comprising (a) a hydrophilic liquid miscible phase, (b) a second hydrophobic phase being immiscible or largely immiscible with the first phase and (c) one or more surfactants, wherein the dispersed phase is in the form of small (e.g. micron to sub-micron diameter, but more usually at least 1 micron diameter) droplets, and the whole having the following characteristics, which distinguish polyaphron dispersions from conventional or common emulsions:
1. They are capable of existing in a stable form wherein the volume fraction of the dispersed phase (φᵢₚ) is greater than 0.7 and can be as high as 0.97. (φᵢₚ is the volume ratio of discontinuous to continuous phase expressed as a fraction).
2. The microscopic appearance of polyaphron dispersions where φᵢₚ is greater than 0.7 of an aggregate of individual droplets, pushed closely together into polyhedral shapes, resembling the appearance of a gas foam. In this form, the dispersion has gel-like properties and is referred to as a Gel Polyaphron Dispersion (GPD).
3. Stable polyaphron dispersions can be formed with a surfactant comprising less than 3% or more typically less than 2% by weight of the total composition.
4. Gel Polyaphron Dispersions (as described in 2 above) can be diluted to any extent by the addition of more continuous phase without the addition of more surfactant, when the gel-like properties disappear. Once φᵢₚ has been reduced to below 0.7, the individual droplets of internal phase become separated to take the form of spherical droplets, which remain stable and intact but which may nevertheless join together in loose associations and float to the top or sink to the bottom of the diluted dispersion (depending on the relative densities of the two phases). In this diluted form each droplet is referred to as a Colloidal Liquid Aphron (CLA). Simple shaking of the diluted dispersion instantly causes a homogeneous, stable dispersion of Colloidal Liquid Aphrons to re-form.
Each of the above characteristics and a combination of them clearly differentiate the polyaphron dispersions of the present invention from conventional emulsions, which have none of those characteristics. Polyaphron dispersions are disclosed in the following literature references by Sebba: "Biliquid Foams", J. Colloid and Interface Science, 40 (1972) 468-474 and "The Behaviour of Minute Oil Droplets Encapsulated in a Water Film", Colloid Polymer Sciences, 257 (1979) 392-396, Hicks "Investigating the Generation, Characterisation, and Structure of Biliquid Foams", PhD Thesis, University of Bristol, 2005, Crutchley "The Encapsulation of Oils and Oil Soluble Substances Within Polymer Films", PhD Thesis, The University of Leeds, 2006 and Lye and Stuckey, Colloid and Surfaces, 131 (1998) 119-136. Aphrons are also disclosed in US-A-4,486,333 and WO 97/32559.

Polyaphron dispersions are sometimes referred to as 'Biliquid Foams', 'High Internal Phase Emulsions (HIPEs)', 'High Internal Phase Ratio Emulsions (HIPREs)' and 'Gel Emulsions'. All such descriptions that refer to dispersions having the characteristics described above are polyaphron dispersions of the present invention.

The present invention will now be further described. In the following passages different aspects of the invention are defined in more detail. Each aspect so defined may be combined with any other aspect or aspects unless clearly indicated to the contrary. In particular any feature indicated as being preferred or advantageous may be combined with any other feature or features indicated as being preferred or advantageous.

As described above polyaphron dispersions comprise a continuous phase, a discontinuous phase and a surfactant. The discontinuous phase is preferably a substantially hydrophobic internal phase, commonly known as an oil internal phase. Preferably, the discontinuous phase comprises a pharmaceutically acceptable oil phase.

Examples of oils which may be used in the present invention include almond oil, babassu oil, blackcurrant seed oil, borage oil, canola oil, castor oil, coconut oil, cod liver oil, corn oil, cottonseed oil, evening primrose oil, fish oil, grapeseed oil, mustard seed oil, oat oil, olive oil, palm kernel oil, palm oil, peanut oil, rapeseed oil, safflower oil, sesame oil, shark liver oil, squalane, soybean oil, sunflower oil, walnut oil, wheat germ oil, hydrogenated castor oil, hydrogenated coconut oil, hydrogenated cottonseed oil, hydrogenated palm oil, hydrogenated soybean oil, partially hydrogenated soybean oil, hydrogenated vegetable oil, isopropyl myristate, isopropyl isostearate, isopropyl palmitate, modified triglycerides, caprylic/capric glycerides, fractionated triglycerides, glyceryl tricaprate, glyceryl tricaproate, glyceryl tricaprylate, glyceryl tricaprylate/caprate, glyceryl tricaprylate/caprate, glyceryl tricaprylate/caprate/laurate, glyceryl tricaprylate/caprate/linoleate, glyceryl tricaprylate/caprate/stearate, glyceryl trilaurate, glyceryl trilinoleate, glyceryl trilinolenate, glyceryl trioleate, glyceryl triundecanoate, linoleic glycerides, saturated polyglycolized glycerides, synthetic medium chain triglyceride containing primarily C₈-C₁₂ fatty acid chains, medium chain triglycerides, long chain triglycerides, modified triglycerides, fractionated triglycerides, and mixtures thereof.

Suitably the discontinuous phase comprises monoglycerides, diglycerides or triglycerides. These may be of particular use when the corticosteroid is at least partially present in the discontinuous phase.

The discontinuous phase may comprise suitable silicone co-solvents, which may be cyclic, linear, or branched chain silicones. Suitable silicones include, but are not limited to cyclomethicone 5-NF, dimethicone 5-NF, or dimethiconol 20.

It will be understood that other suitable oils may be used in the present invention.

The discontinuous phase may, for example, confer an emollient, occlusive, moisturising, conditioning or other cosmetic or pharmaceutical benefit to the skin. It may also increase the viscosity of the composition and may confer solvency to the active.

The composition may comprise at least 1 % by weight of the discontinuous phase, preferably from 1 to 80% by weight, more preferably from 10% to 80% by weight and most preferably from 25% to 80% by weight based on the total weight of the total composition.

The continuous or external phase of the polyaphron dispersion is preferably formed of a hydrophilic liquid, and is preferably substantially non-aqueous. The continuous phase preferably comprises from 20 to 99 wt%, more preferably from 20 to 75 wt% and most preferably from 50 to 75 wt% water based on the total weight of the composition. When any part of the continuous phase is aqueous, close control of the pH within suitable limits is essential as would be understood by those skilled in the art.

The continuous phase may comprise or consist of water, a pharmaceutically acceptable liquid that is miscible or substantially miscible with water or a mixture thereof. The water miscible liquid is preferably a compound of formula R₁-OH where R₁ is C₁-C₁₀ alkyl and/or a compound of formula HO-R₂-H where R₂ is (C₂H₄)ₙ or (C₃H₆)ₙ where n is 1 to 100, preferably 1 to 25. R₁ and R₂ may be linear or branched. Preferably R₁ is C₁-C₄ alkyl. n is preferably 1 to 25. Preferably the continuous phase comprises water, propylene glycol or polypropylene glycol, ethylene glycol or polyethylene glycol, glycerol, ethanol, or a mixture thereof. More preferably the continuous phase comprises diethylene glycol mono ethyl ether, or other suitable ether derivatives.

Where the continuous phase comprises polyethylene glycol, the polyethylene glycol is preferably a polyethylene glycol which is liquid at room temperature. The polyethylene glycol may, for example, contain from 1 to 12 ethylene or propylene oxide units and/or have a molecular weight of up to 600.

The continuous phase comprises from 0 to 60 wt%, preferably from 0 to 20 wt%, more preferably from 0 to 10 wt%, of a C₁ - C₄ alcohol, ethylene glycol, a liquid polyethylene glycol, propylene glycol, a liquid polypropylene glycol, diethylene glycol mono ethyl ether or mixtures thereof. One of the advantages of the composition of the present invention over prior art compositions is that high levels of alcohol are not needed as a skin permeation enhancers. Alcohol is known to cause skin irritation when applied to damaged skin. The compositions of the present invention have the advantage that enhanced permeation can be achieved even without the need for alcohol to be present.

It will be understood that other suitable hydrophobic solvents may be used in the continuous phase of the polyaphrons.

The surfactant used in the present invention may be incorporated into either or both phases of the polyaphron dispersion. Suitable surfactants include an alkyl polyglycol ether, an alkyl polyglycol ester, an ethoxylated alcohol, a polyoxyethylene sorbitan fatty acid ester, a polyoxyethylene fatty acid ester, an ionic or non-ionic surfactant, a hydrogenated castor oil/polyoxyethylene glycol adducts containing from 25 to 60 ethoxy groups a castor oil/polyoxyethylene glycol adduct containing from 25 to 45 ethoxy groups, a sorbitan fatty acid ester (for example Span 20 or Span 80), a block copolymer of ethylene oxide and propylene oxide (for example Pluronic L121 or Pluronic F68), or a mixture thereof.

It will be understood that other suitable surfactants may be used.

Preferably the compositions of the present invention comprise less than 4% by weight of surfactant, more preferably less than 3% by weight, more preferably still less than 2% by weight of the total composition.

The corticosteroid may be predominantly in the continuous phase, or predominantly in the discontinuous phase. Preferably, the corticosteroid is predominantly in the continuous phase.

Preferably, the corticosteroid is selected from one or more of Betamethasone (9 -fluoro-11 ,17 ,21-trihydroxy-16 - methylpregna-1,4-diene-3,20-dione) or esters thereof such as the 21-acetate, 17-adamantoate, 17-benzoate, 17-valerate, and 17,21-dipropionate; Alclomethasone or esters thereof such as the 17,21-dipropionate; Clobetasole or esters thereof such as the propionate; Clobetasone or esters thereof such as the 17-butyrate; Desoximetasone; Diflucortolon or esters thereof, Diaflorasone or esters thereof such as the 17,21-diacetate; Fluocinonid; Flumetasone or esters thereof such as the 21-pivalate; Fluocinolone or ethers thereof such as the acetonide; Fluticasone or ester thereof such as the 17-propionate; Fluprednidene or esters thereof such as the 21-acetate; Halcinonide; Hydrocortisone or esters thereof such as the 17-butyrate; Mometasone or esters thereof such as the 17-(2-furoate); and Triamcinolon or ethers or esters thereof such as the acetonide. More preferably the corticosteroid is selected from one or more of Betamethasone or esters thereof such as the 17-valerate or the 17,21-dipropionate; Clobetasole or esters thereof such as the propionate; Triamcinolon or ethers or esters thereof such as the acetonide or the acetonide-21-N-benzoyl-2-methyl- - alaninate or the acetonide-21-(3,3-dimethylbutyrate); or Hydrocortisone or esters thereof such as the 17-butyrate. Most preferably, the corticosteroid is betamethasone.

The composition of the present invention preferably comprises from 0.001 to 1.0% by weight of corticosteroid, more preferably from 0.01% to 0.075% by weight and more preferably still from 0.025 to 0.05% by weight of the total composition.

The composition of the present invention may further comprise a gelling agent and/or a rheology modifying agent, such as a viscosity modifier.

The composition of the present invention may further comprise a gelling agent and/or a viscosity modifying agent such as a rheology modifying agent.

The gelling agent may, for example, be selected from alginate gums or their salts, guar gum, locust bean gum, xanthan gum, gum acacia, gelatin, carrageenans, hydroxymethyl-cellulose hydroxyethylcellulose, hydroxypropyl-cellulose, carboxymethylcellulose or its salts, bentonites, magnesium aluminium silicates, "Carbomers" (salts of cross-linked polymers of acrylic acid), or glyceryl polymethacrylates or their dispersions in glycols.

Preferably, the composition of the present invention comprises from 0.05 to 5.0% by weight of a gelling agent, preferably from 0.1 to 2.0% by weight and more preferably from 0.2 to 1.0% by weight of the total composition.

The composition of the present invention may be used in a method of treatment of the human or animal body by therapy. Further, the composition of the present invention may be used in the treatment of radiation dermatitis, eczema, rheumatoid arthritis, and/or psoriasis. Also the composition of the present invention may be used in the manufacture of a medicament for treatment of radiation dermatitis, eczema, rheumatoid arthritis, and/or psoriasis.

The compositions of the present invention may also contain other additives such as preservatives (for instance to prevent microbiological spoilage), buffering agents (for the control of pH and to avoid instability and damage to the skins acid mantel), antioxidants and permeation enhancers. These additives may be included in the continuous or the discontinuous phase of the polyaphron dispersion.

It will be understood that the inclusion of these additives will be at the levels and with the type of materials which are found to be effective and useful. Care needs to be taken in the choice and amount of these additives to prevent compromise to the other performance advantages of the present invention.

The composition of the present invention may further comprise additional active agents, for example vitamin D or a vitamin D analogue. Desirably the composition does not contain vitamin D or vitamin D analogues.

In one embodiment the discontinuous phase is a caprylic/capric triglyceride, the continuous phase is demineralised water and the corticosteroid is a betamethasone.

Accordingly to one aspect of the present invention, there is provided a method of making the composition as described herein comprising the following steps:
(i) providing a hydrophilic solvent, optionally comprising at least one corticosteroid, and/or a surfactant;
(ii) providing a hydrophobic solvent optionally comprising at least one corticosteroid, and/or a surfactant;
(iii) mixing the hydrophilic solvent with the hydrophobic solvent under suitable conditions to form the composition comprising at least one polyaphron dispersion and at least one corticosteroid.

In one embodiment of the present invention the corticosteroid is passed into the hydrophobic solvent prior to the polyaphron formation step. Alternately, or additionally, the corticosteroid may be mixed in to the continuous phase prior to the polyaphron formation step.

Suitable methods for preparing polyaphron dispersions are described in US-A-4486333. It will be understood by those skilled in the art that other manufacturing methods may be used, as appropriate.

The following Examples further illustrate the present invention.

### Gel Polyaphron Disperson Preparation

### Method of Manufacture of the Gel Polyaphron dispersion

A low form, 250ml laboratory beaker (internal diameter 6.5cm) was charged with sufficient aqueous (continuous) phase to make 30 g of gel polyaphron. This was stirred at 200 rpm with a four-bladed impeller having a diameter of 6.0 cm whilst adding the oil (discontinuous) phase dropwise from a Pasteur pipette. The rate of addition at the start of the process was slow (approximately one drop every 7 seconds) but was speeded up once 20% of the oil phase had been added so that the total time to make the gel polyaphron was approximately 20 minutes.

The following dispersions were prepared.

| ***GEL POLYAPHRON DISPERSION 1*** | |
|---|---|
| ***Oil Phase*** | **%** |
| isopropyl isostearate (oil) | 10.00 |
| caprylic capric triglyceride (oil) (Waglinol 3/9280 ex Paroxite) | 7.5 |
| Cetearyl octanoate | 7.5 |
| Dimethiconol 20 | 20.00 |
| ST cyclomethicone 5 NF | 15.00 |
| Silicone fluid DC 200 100 centistokes | 29.00 |
| Laureth-4 (Volpo L4 ex Croda :C12-C13 pareth- 4) | 1.00 |
| | |
| | |

| ***Aqueous Phase*** | |
|---|---|
| sodium lauryl ether sulphate :Texapon NSO/IS (surfactant) | 0.10 |
| Demineralised Water | 9.90 |
| | |

| ***GEL POLYAPHRON DISPERSION 2*** | |
|---|---|
| ***Oil Phase*** | **%** |
| isopropyl isostearate (oil) | 10.00 |
| caprylic capric triglyceride (oil) (Waglinol 3/9280 ex Paroxite) | 7.5 |
| Squalane | 7.5 |
| Dimethiconol 20 | 20.00 |
| ST cyclomethicone 5-NF | 15.00 |
| Silicone fluid DC 200 100 centistokes | 29.00 |
| Laureth-4 (Volpo L4 ex Croda :C12-C13 pareth- 4) | 1.00 |

| ***Aqueous Phase*** | |
|---|---|
| Tween 20 (polysorbate 20 ex Surfachem) | 0.12 |
| Demineralised Water | 9.88 |

| ***GEL POLYAPHRON DISPERSION 3*** | |
|---|---|
| ***Oil Phase*** | **%** |
| isopropyl myristate (oil) | 10.00 |
| caprylic capric triglyceride (oil) (Waglinol 3/9280 ex Paroxite) | 7.3 |
| Squalane | 7.5 |
| Dimethiconol 20 | 20.00 |
| ST cyclomethicone 5-NF | 15.00 |
| Silicone fluid DC 200 100 centistokes | 29.00 |
| Laureth-4 (Volpo L4 ex Croda :C12-C13 pareth- 4) | 1.2 |
| | |
| | |

| ***Aqueous Phase*** | |
|---|---|
| Tween 20 (polysorbate 20 ex Surfachem) | 0.12 |
| Demineralised Water | 9.88 |

| ***GEL POLYAPHRON DISPERSION 4*** | |
|---|---|
| ***Oil Phase*** | **%** |
| isopropyl myristate (oil) | 10.00 |
| caprylic capric triglyceride (oil) (Waglinol 3/9280 ex Paroxite) | 6.7 |
| Squalane | 6.9 |
| Dimethiconol 20 | 20.00 |
| ST cyclomethicone 5-NF | 15.00 |
| Silicone fluid DC 200 100 centistokes | 29.00 |
| Laureth-4 (Volpo L4 ex Croda :C12-C13 pareth- 4) | 2.4 |
| | |
| | |

| ***Aqueous Phase*** | |
|---|---|
| Tween 20 (polysorbate 20 ex Surfachem) | 0.12 |
| Demineralised Water | 9.88 |

| ***POLYAPHRON DISPERSION 5*** | |
|---|---|
| ***Oil Phase*** | **%** |
| isopropyl myristate (oil) | 10.00 |
| caprylic capric triglyceride (oil) (Waglinol 3/9280 ex Paroxite) | 6.7 |
| Squalane | 6.9 |
| Dimethiconol 20 | 19.40 |
| ST cyclomethicone 5-NF | 15.00 |
| Silicone fluid DC 200 100 centistokes | 28.40 |
| Laureth-4 (Volpo L4 ex Croda :C12-C13 pareth- 4) | 3.60 |
| | |
| | |

| ***Aqueous Phase*** | |
|---|---|
| Tween 20 :polysorbate 20 ex Surfachem | 0.12 |
| Demineralised Water | 9.88 |

| ***GEL POLYAPHRON DISPERSION 6*** | |
|---|---|
| ***Oil Phase*** | **%** |
| isopropyl myristate (oil) | 10.00 |
| caprylic capric triglyceride (oil) (Waglinol 3/9280 ex Paroxite) | 6.7 |
| Squalane | 6.9 |
| Dimethiconol 20 | 18.80 |
| ST cyclomethicone 5-NF | 15.00 |
| Silicone fluid DC 200 100 centistokes | 27.80 |
| Laureth-4 (Volpo L4 ex Croda :C12-C13 pareth- 4) | 4.8 |
| | |
| | |

| ***Aqueous Phase*** | |
|---|---|
| Tween 20 :polysorbate 20 ex Surfachem | 0.12 |
| Demineralised Water | 9.88 |

| ***GEL POLYAPHRON DISPERSION 7*** | |
|---|---|
| ***Oil Phase*** | **%** |
| isopropyl myristate (oil) | 10.00 |
| caprylic capric triglyceride (oil) (Waglinol 3/9280 ex Paroxite) | 6.70 |
| Squalane | 6.90 |
| Dimethiconol 20 | 17.60 |
| ST cyclomethicone 5-NF | 14.00 |
| Silicone fluid DC 200 100 centistokes | 26.80 |
| Laureth-4 (Volpo L4 ex Croda :C12-C13 pareth- 4) | 8.00 |
| | |
| | |

| ***Aqueous Phase*** | |
|---|---|
| Tween 20 :polysorbate 20 ex Surfachem | 0.12 |
| Demineralised Water | 9.88 |

| ***GEL POLYAPHRON DISPERSION 8*** | |
|---|---|
| ***Oil Phase*** | **%** |
| isopropyl myristate (oil) | 10.42 |
| caprylic capric triglyceride (oil) (Waglinol 3/9280 ex Paroxite) | 6.98 |
| Squalane | 7.18 |
| Dimethiconol 20 | 17.29 |
| ST cyclomethicone 5-NF | 13.54 |
| Silicone fluid DC 200 100 centistokes | 25.83 |
| Laureth-4 (Volpo L4 ex Croda :C12-C13 pareth- 4) | 8.33 |
| | |
| | |

| ***Aqueous Phase*** | |
|---|---|
| Tween 20 :polysorbate 20 ex Surfachem | 0.13 |
| Demineralised Water | 10.30 |

| ***GEL POLYAPHRON DISPERSION 9*** | |
|---|---|
| ***Oil Phase*** | **%** |
| Castor oil | 30.00 |
| caprylic capric triglyceride (oil) (Waglinol 3/9280 ex Paroxite) | 48.00 |
| isopropyl myristate (oil) (ex Inolex Chemicals) | 5.70 |
| Tween 80 : polysorbate 80 ex Surfachem | 0.90 |
| DL-menthol | 1.30 |
| Vitamin E | 4.00 |
| Betamethasone dipropionate (BDP) | 0.10 |
| | |
| | |

| ***Aqueous Phase*** | |
|---|---|
| Tween 80 : polysorbate 80 ex Surfachem | 0.10 |
| Demineralised Water | 9.90 |

| ***GEL POLYAPHRON DISPERSION 10*** | |
|---|---|
| ***Oil Phase*** | **%** |
| isopropyl myristate (oil) | 10.00 |
| caprylic capric triglyceride (oil) (Waglinol 3/9280 ex Paroxite) | 15.00 |
| Squalane | 19.10 |
| Elastomer DC10 | 20.00 |
| Silicone fluid DC 200 100 centistokes | 25.00 |
| Laureth-4 (Volpo L4 ex Croda :C12-C13 pareth- 4) | 0.90 |
| | |
| | |

| ***Aqueous Phase*** | |
|---|---|
| sodium lauryl ether sulphate :Texapon NSO/IS (surfactant) | 0.10 |
| Demineralised Water | 9.90 |

| ***GEL POLYAPHRON DISPERSION 11*** | |
|---|---|
| ***Oil Phase*** | **%** |
| isopropyl myristate (oil) | 10.00 |
| caprylic capric triglyceride (oil) (Waglinol 3/9280 ex Paroxite) | 7.3 |
| Squalane | 7.5 |
| Dimethiconol 20 | 20.0 |
| ST cyclomethicone 5-NF | 15.00 |
| Silicone fluid DC 200 100 centistokes | 29.0 |
| Laureth-4 (Volpo L4 ex Croda :C12-C13 pareth- 4) | 1.2 |
| | |
| | |

| ***Aqueous Phase*** | |
|---|---|
| Tween 20 :polysorbate 20 ex Surfachem (surfactant) | 0.12 |
| Demineralised Water | 9.88 |

| ***GEL POLYAPHRON DISPERSION 12*** | |
|---|---|
| ***Oil Phase*** | **%** |
| isopropyl myristate (oil) | 10.00 |
| caprylic capric triglyceride (oil) (Waglinol 3/9280 ex Paroxite) | 6.70 |
| Squalane | 6.90 |
| Dimethiconol 20 | 20.00 |
| ST cyclomethicone 5-NF | 15.00 |
| Silicone fluid DC 200 100 centistokes | 29.00 |
| Laureth-4 (Volpo L4 ex Croda :C12-C13 pareth- 4) | 2.40 |
| | |
| | |

| ***Aqueous Phase*** | |
|---|---|
| Tween 20 :polysorbate 20 ex Surfachem (surfactant) | 0.12 |
| Demineralised Water | 9.88 |

| ***GEL POLYAPHRON DISPERSION 13*** | |
|---|---|
| ***Oil Phase*** | **%** |
| isopropyl myristate (oil) | 10.00 |
| caprylic capric triglyceride (oil) (Waglinol 3/9280 ex Paroxite) | 6.7 |
| Squalane | 6.9 |
| Dimethiconol 20 | 18.80 |
| ST cyclomethicone 5-NF | 15.00 |
| Silicone fluid DC 200 100 centistokes | 27.80 |
| Laureth-4 (Volpo L4 ex Croda :C12-C13 pareth- 4) | 4.80 |
| | |
| | |

| ***Aqueous Phase*** | |
|---|---|
| Tween 20 :polysorbate 20 ex Surfachem (surfactant) | 0.12 |
| Demineralised Water | 9.88 |

| ***GEL POLYAPHRON DISPERSION 14*** | |
|---|---|
| ***Oil Phase*** | **%** |
| isopropyl myristate (oil) | 10.00 |
| caprylic capric triglyceride (oil) (Waglinol 3/9280 ex Paroxite) | 6.7 |
| Squalane | 6.9 |
| Dimethiconol 20 | 17.60 |
| ST cyclomethicone 5-NF | 14.00 |
| Silicone fluid DC 200 100 centistokes | 26.80 |
| Laureth-4 (Volpo L4 ex Croda :C12-C13 pareth- 4) | 8.00 |
| | |
| | |

| ***Aqueous Phase*** | |
|---|---|
| Tween 20 :polysorbate 20 ex Surfachem (surfactant) | 0.12 |
| Demineralised Water | 9.88 |

| ***GEL POLYAPHRON DISPERSION 15*** | |
|---|---|
| ***Oil Phase*** | **%** |
| Castor oil | 27.00 |
| caprylic capric triglyceride (oil) (Waglinol 3/9280 ex Paroxite) | 55.213 |
| Tween 80 : polysorbate 80 ex Surfachem (surfactant) | 0.90 |
| DL-menthol | 1.35 |
| Retinol palmitate | 0.297 |
| 2,6-Di-tert butyl 4-methylphenol (antioxidant) | 0.137 |
| Vitamin E acetate | 5.04 |
| Dexamethasone acetate | 0.063 |
| | |

| ***Aqueous Phase*** | |
|---|---|
| Tween 80 : polysorbate 80 ex Surfachem (surfactant) | 0.12 |
| Demineralised Water | 9.88 |
| | |

| ***GEL POLYAPHRON DISPERSION 16*** | |
|---|---|
| ***Oil Phase*** | **%** |
| Cetearyl isononoate | 30.00 |
| Isopar K (ex Exxon Chemical Limited (oil)) | 30.00 |
| DC 245 | 15.00 |
| Silicone fluid DC 200 100 centistokes | 14.00 |
| Laureth-4 (Volpo L4 ex Croda :C12-C13 pareth- 4) | 1.00 |
| | |
| | |

| ***Aqueous Phase*** | |
|---|---|
| Demineralised Water | 10.00 |

### Example preparation for corticosteroids

The carbomer was dispersed in water using a high shear rotastator mixer (Silverson). The carbomer was fully dispersed and an aqueous solution of 50% triethylamine in water was added until a clear viscous gel at pH 7 was obtained. Meanwhile, the active (when not present only in the concentrated polyaphron dispersion) was dissolved in the water miscible solvents (usually propylene glycol and Transcutol P (diethylene glycol monoethyl ether EP ex Gattefosse)) by addition and mixing. The solution of the active was added to the carbomer gel with stirring. The concentrated polyaphron dispersion was then mixed with the polymer gel until a semi-viscous white gel formulation was obtained. All processes were carried out at room temperature.

In the examples of the present invention:
Natrasol 250HR is hydroxyethyl cellulose from Hercules
Euxyl 400 is from Schulke & Mayr

| ***Example 1 Hydrocortisone acetate 0.05%w*/*w AGED 2 YEARS*** | |
|---|---|
| | **%** |
| White soft paraffin | 8 |
| Crothix liquid (PEG-150 pentaerythrityl tetrastearate) | 2 |
| Sepigel 305 ex Seppic | 2 |
| | |
| | |
| Propylene glycol | 10 |
| Transcutol P (diethylene glycol monoethyl ether EP ex Gattefosse) | 10 |
| Hydrocortisone acetate | 0.05 |
| | |
| Demineralised water | 42.85 |
| Euxyl K400 | 0.1 |
| Gel polyaphron dispersion 1 | 25 |

| ***Example 2 Hydrocortisone* acetate *0.05%w*/*w FRESH*** | |
|---|---|
| | **%** |
| White soft paraffin | 8 |
| Crothix liquid (PEG-150 pentaerythrityl tetrastearate) | 2 |
| Sepigel 305 ex Seppic | 2 |
| | |
| | |
| Propylene glycol | 10 |
| Transcutol P (diethylene glycol monoethyl ether EP ex Gattefosse) | 10 |
| Hydrocortisone acetate | 0.05 |
| | |
| Demineralised water | 42.85 |
| Euxyl K400 | 0.1 |
| Gel polyaphron dispersion 1 | 25 |

| ***Example 3 Hydrocortisone acetate 0.05%w*/*w*** | |
|---|---|
| | **%** |
| White soft paraffin | 8 |
| Simulgel 600 PHA ex Seppic | 2 |
| Natrasol solution in demineralised water (2%w/w) | 10 |
| | |
| | |
| Propylene glycol | 10 |
| Transcutol P (diethylene glycol monoethyl ether EP ex Gattefosse) | 10 |
| Hydrocortisone acetate | 0.05 |
| | |
| Demineralised water | 34.85 |
| Bronopol preservative (2-bromo-2-nitropropane-1,3-diol) | 0.1 |
| Gel polyaphron dispersion 2 | 25 |

| ***Example 4 Hydrocortisone acetate 0.05%w*/*w*** | |
|---|---|
| | **%** |
| White soft paraffin | 8 |
| Simulgel 600 PHA ex Seppic | 2 |
| Natrasol solution in demineralised water (2%w/w) | 10 |
| | |
| | |
| Propylene glycol | 10 |
| Transcutol P (diethylene glycol monoethyl ether EP ex Gattefosse) | 10 |
| Hydrocortisone acetate | 0.05 |
| | |
| Demineralised water | 34.85 |
| Bronopol preservative | 0.1 |
| Gel polyaphron dispersion 3 | 25 |

| ***Example 5 Hydrocortisone acetate 0.05%w*/*w*** | |
|---|---|
| | **%** |
| White soft paraffin | 8 |
| Simulgel 600 PHA ex Seppic | 2 |
| Natrasol solution in demineralised water (2%w/w) | 10 |
| | |
| | |
| Propylene glycol | 10 |
| Transcutol P (diethylene glycol monoethyl ether EP ex Gattefosse) | 10 |
| Hydrocortisone acetate | 0.05 |
| | |
| Demineralised water | 34.85 |
| Bronopol preservative (2-bromo-2-nitropropane-1,3-diol) | 0.1 |
| Gel polyaphron dispersion 4 | 25 |

| ***Example 6 Hydrocortisone acetate 0.05%w*/*w*** | |
|---|---|
| | **%** |
| White soft paraffin | 8 |
| Simulgel 600 PHA ex Seppic | 2 |
| Natrasol solution in demineralised water (2%w/w) | 10 |
| | |
| | |
| Propylene glycol | 10 |
| Transcutol P (diethylene glycol monoethyl ether EP ex Gattefosse) | 10 |
| Hydrocortisone acetate | 0.05 |
| | |
| Demineralised water | 34.85 |
| Bronopol preservative (2-bromo-2-nitropropane-1,3-diol) | 0.1 |
| Gel polyaphron dispersion 5 | 25 |

| ***Example 7 Hydrocortisone acetate 0.05%w*/*w*** | |
|---|---|
| | **%** |
| White soft paraffin | 8 |
| Simulgel 600 PHA ex Seppic | 2 |
| Natrasol solution in demineralised water (2%w/w) | 10 |
| | |
| | |
| Propylene glycol | 10 |
| Transcutol P (diethylene glycol monoethyl ether EP ex Gattefosse) | 10 |
| Hydrocortisone acetate | 0.05 |
| | |
| Demineralised water | 34.85 |
| Bronopol preservative (2-bromo-2-nitropropane-1,3-diol) | 0.1 |
| Gel polyaphron dispersion 6 | 25 |

| ***Example 8 Hydrocortisone acetate 0.05%w*/*w*** | |
|---|---|
| | **%** |
| White soft paraffin | 8 |
| Simulgel 600 PHA ex Seppic | 2 |
| Natrasol solution in demineralised water (2%w/w) | 10 |
| | |
| | |
| Propylene glycol | 10 |
| Transcutol P (diethylene glycol monoethyl ether EP ex Gattefosse) | 10 |
| Hydrocortisone acetate | 0.05 |
| | |
| Demineralised water | 34.85 |
| Bronopol preservative (2-bromo-2-nitropropane-1,3-diol) | 0.1 |
| Gel polyaphron dispersion 7 | 25 |

| ***Example 9 Hydrocortisone acetate 0.05%w*/*w*** | |
|---|---|
| | **%** |
| White soft paraffin | 8 |
| Simulgel 600 PHA ex Seppic | 2 |
| Natrasol solution in demineralised water (2%w/w) | 10 |
| Laureth-4 (Volpo L4 - Croda) | 1 |
| | |
| Propylene glycol | 10 |
| Transcutol P (diethylene glycol monoethyl ether EP ex Gattefosse) | 10 |
| Hydrocortisone acetate | 0.05 |
| | |
| Demineralised water | 33.85 |
| Bronopol preservative (2-bromo-2-nitropropane-1,3-diol) | 0.1 |
| Gel polyaphron dispersion 8 | 25 |

| ***Example 10 Betamethasone dipropionate 0.03%w*/*w*** | |
|---|---|
| | **%** |
| White soft paraffin | 8 |
| Simulgel 600 PHA ex Seppic | 2 |
| Natrasol solution in demineralised water (2%w/w) | 10 |
| | |
| Propylene glycol | 2.5 |
| | |
| Demineralised water | 37.4 |
| Bronopol preservative (2-bromo-2-nitropropane-1,3-diol) | 0.1 |
| Gel polyaphron dispersion 9 | 30 |
| Gel polyaphron dispersion 10 | 10 |

| ***Example 11 Betamethasone dipropionate 0.0643%w*/*w*** | |
|---|---|
| | **%** |
| White soft paraffin | 8 |
| Simulgel 600 PHA ex Seppic | 2 |
| Natrasol solution in demineralised water (2%w/w) | 10 |
| | |
| Propylene glycol | 10 |
| Transcutol P (diethylene glycol monoethyl ether EP ex Gattefosse) | 10 |
| Betamethasone dipropionate | 0.0643 |
| | |
| Demineralised water | 34.8357 |
| Bronopol preservative (2-bromo-2-nitropropane-1,3-diol) | 0.1 |
| Gel polyaphron dispersion 11 | 25 |

| ***Example 12 Betamethasone dipropionate 0.0643%w*/*w*** | |
|---|---|
| | **%** |
| White soft paraffin | 8 |
| Simulgel 600 PHA ex Seppic | 2 |
| Natrasol solution in demineralised water (2%w/w) | 10 |
| | |
| Propylene glycol | 10 |
| Transcutol P (diethylene glycol monoethyl ether EP ex Gattefosse) | 10 |
| Betamethasone dipropionate | 0.0643 |
| | |
| Demineralised water | 34.8357 |
| Bronopol preservative (2-bromo-2-nitropropane-1,3-diol) | 0.1 |
| Gel polyaphron dispersion 12 | 25 |

| ***Example 13 Betamethasone dipropionate 0.0643%w*/*w*** | |
|---|---|
| | **%** |
| White soft paraffin | 8 |
| Simulgel 600 PHA ex Seppic | 2 |
| Natrasol solution in demineralised water (2%w/w) | 10 |
| | |
| Propylene glycol | 10 |
| Transcutol P (diethylene glycol monoethyl ether EP ex Gattefosse) | 10 |
| Betamethasone dipropionate | 0.0643 |
| | |
| Demineralised water | 34.8357 |
| Bronopol preservative (2-bromo-2-nitropropane-1,3-diol) | 0.1 |
| Gel polyaphron dispersion 13 | 25 |

| ***Example 14 Betamethasone dipropionate 0.0643%w*/*w*** | |
|---|---|
| | **%** |
| White soft paraffin | 8 |
| Simulgel 600 PHA ex Seppic | 2 |
| Natrasol solution in demineralised water (2%w/w) | 10 |
| | |
| Propylene glycol | 10 |
| Transcutol P (diethylene glycol monoethyl ether EP ex Gattefosse) | 10 |
| Betamethasone dipropionate | 0.0643 |
| | |
| Demineralised water | 34.8357 |
| Bronopol preservative (2-bromo-2-nitropropane-1,3-diol) | 0.1 |
| Gel polyaphron dispersion 14 | 25 |

| ***Example 15 Betamethasone dipropionate 0.0643%w*/*w*** | |
|---|---|
| | **%** |
| White soft paraffin | 8 |
| Simulgel 600 PHA ex Seppic | 2 |
| Natrasol solution in demineralised water (2%w/w) | 10 |
| Laureth-4 (Volpo L4 - Croda) | 1 |
| | |
| Propylene glycol | 10 |
| Transcutol P (diethylene glycol monoethyl ether EP ex Gattefosse) | 10 |
| Betamethasone dipropionate | 0.0643 |
| | |
| Demineralised water | 33.8357 |
| Bronopol preservative (2-bromo-2-nitropropane-1,3-diol) | 0.1 |
| Gel polyaphron dispersion 8 | 25 |

| ***Example 16 Dexamethasone acetate 0.025% w*/*w*** | |
|---|---|
| | **%** |
| Carbomer 980 (1%w/w) in demineralised water neutralised with TEA | 34.9 |
| Natrasol solution in demineralised water (2%w/w) | 8 |
| | |
| Sodium hyaluronate (1%w/w) in demineralised water | 5 |
| Propylene glycol | 2 |
| Thymol | 0.1 |
| | |
| Gel polyaphron dispersion 15 | 40 |
| Gel polyaphron dispersion 16 | 10 |

The composition of Examples 1, 2 and 3 were tested for steady state of corticosteroid flux rate (measured over 8 hours) through silicone membrane in Franz diffusion cells. This is a recognised in vitro test correlating to permeation through the skin. For comparison the commercial products Hc45 and Dermacort were also tested.

**Table 1 Steady State Flux Rate of Hydrocortisone Acetate(HA) through Silicone Membrane in Franz Diffusion Cells**

| **Composite** | **Hydrocortisone Acetate (HA)** | **Flux Rate ngcm⁻² hr⁻¹** | **Flux Rate per % of HA content** | **Ratio of Sample Flux to Hc45 per %** |
|---|---|---|---|---|
| Example 1 | 0.05 | 302.38 | 6048 | 25.2 |
| Example 2 | 0.05 | 305.00 | 6100 | 25.4 |
| Example 3 | 0.05 | 282.63 | 5653 | 23.6 |
| Hc45 | 1.0 | 240.38 | 240 | 1.0 |
| Dermacort | 0.1 | 113.50 | 1135 | 4.7 |

**Table 2 - Hydrocortisone Acetate (HA) Diffusion Study. Relationship between Steady state Flux Rate v Surfactant Concentration (Laureth - 4) through Silicone Membrane in Franz Diffusion Cells for HA.**

| Example Number | 4 | 5 | 6 | 7 | 8 | 9 |
|---|---|---|---|---|---|---|
| Surfactant Concentration (% w/w) | 0.3 | 0.6 | 0.9 | 1.2 | 2.0 | 3.0 |
| Steady State Mean Flux Rate (ng cm ⁻² hr⁻¹⁾ | 317 | 352 | 350 | 412 | 367 | 91 |
| Ratio of mean to rate at 3% | 3.5 | 3.9 | 3.8 | 4.5 | 4.0 | 1.0 |

**Table 3 - Betamethasone Dipropionate (BDP) Diffusion Study Relationship between Steady state Flux Rate v Surfactant Concentration (Laureth - 4) through Silicone Membrane in Franz Diffusion Cells for BDP.**

| Example Number | 11 | 12 | 13 | 14 | 15 |
|---|---|---|---|---|---|
| Surfactant Concentration (% w/w) | 0.3 | 0.6 | 1.2 | 2.0 | 3.0 |
| Steady State Mean Flux Rate (ng cm ⁻² hr⁻¹⁾ | 1270 | 1310 | 1500 | 1180 | 1000 |
| Ratio of mean to rate at 3% | 1.3 | 1.3 | 1.5 | 1.2 | 1.0 |

The data in Table 1 shows the greater flux rate for the composition of the invention as compared to the prior art corrected for identical corticosteroid content.

The data in Tables 2 and 3 show the relationship between surfactant content and flux rate showing that the flux rate can be optimised by contracting the surfactant content.

## Claims

1. A topical composition comprising a continuous phase and at least one discontinuous phase, said composition comprising at least one polyaphron dispersion and at least one corticosteroid and said continuous phase comprising from 0 to 60% by weight of a C₁ - C₄ alcohol, ethylene glycol, a liquid polyethylene glycol, propylene glycol, a liquid polypropylene glycol, diethylene glycol mono ethyl ether or mixtures thereof.

2. A composition according to claim 1 wherein the continuous phase comprises from 0% to 20% by weight of a C₁ - C₄ alcohol, ethylene glycol, a liquid polyethylene glycol, propylene glycol, a liquid polypropylene glycol, diethylene glycol mono ethyl ether or mixtures thereof.

3. A composition according to claim 1 or 2 wherein the corticosteroid is betamethasone, clobetasol, clobetasone, desoximethasone, diflucortolon, difluorasone, fluocinoid, flumethasone, fluocinolon, fluticasone, fluprednidene, halcinonide, hydrocortisone, momethasone, triamcinolon and their esters, or a mixture thereof.

4. A composition according to claim 3 wherein the corticosteroid is betamethasone or an ester thereof.

5. A composition according to any one of the preceding claims which comprises from 0.001 to 1.0% by weight of corticosteroid.

6. A composition according to any one of the preceding claims wherein the discontinuous phase comprises an oil.

7. A composition according to claim 6 wherein the oil comprises a monoglyceride, diglyceride or triglyceride or a mixture thereof.

8. A composition according to any one of the preceding claims comprising at least 20% by weight of the continuous phase based on the total weight of the composition.

9. A composition according to any one of the preceding claims wherein the continuous phase comprises from 20 to 99% by weight of water based on the total weight of the composition.

10. A composition according to claim 9 wherein the continuous phase comprises at least 25% by weight of water based on the total weight of the composition.

11. A composition according to any one of the preceding claims wherein the continuous phase comprises a compound of formula R₁-OH where R₁ is C₁-C₁₀ alkyl and/or a compound of formula HO-R₂-H where R2 is (C₂H₄)n or (C₃H₆)n where n is 1 to 10 or a mixture thereof.

12. A composition according to any one of the preceding claims further comprising a gelling agent.

13. A composition according to any one of the preceding claims further comprising a permeation enhancer.

14. A composition according to any one of the preceding claims which does not comprise vitamin D or a vitamin D analogue.

15. A composition as defined in any one of claims 1 to 14 for use in a method of treatment of the human or animal body by therapy, in particular by topical application.

16. A composition as defined in any one of claims 1 to 14 for use in the topical treatment of radiation dermatitis.

17. A composition as defined in any one of claims 1 to 14 for use in the topical treatment of eczema.

18. A composition as defined in any one of claims 1 to 14 for use in the topical treatment of rheumatoid arthritis.

19. Use of a composition as defined in any one of claims 1 to 14 in the manufacture of a medicament for the topical treatment of radiation dermatitis.

20. Use of a composition as defined in any one of claims 1 to 14 in the manufacture of a medicament for the topical treatment of eczema.

21. Use of a composition as defined in any one of claims 1 to 14 in the manufacture of a medicament for the topical treatment of rheumatoid arthritis.

22. A method of making the composition as defined in any one of claims 1 to 14 comprising the following steps:
(i) providing a hydrophilic solvent, optionally comprising at least one corticosteroid, and/or a surfactant;
(ii) providing a hydrophobic solvent optionally comprising at least one corticosteroid, and/or a surfactant;
(iii)mixing the hydrophilic solvent with the hydrophobic solvent under suitable conditions to form the composition comprising at least one polyaphron dispersion and at least one corticosteroid.
